# EUROPEAN PATENT APPLICATION

(11) **EP 3 753 944 A1**
(43) Date of publication of application: **23.12.2020**
(21) Application number: 19180438.4
(22) Date of filing: 17.06.2019
(51) Int. Cl.: C07F 9/54, A61K 31/4196, C07D 249/08

(54) **3,5-BIS(PHENYL)-1H-HETEROARYL DERIVATIVES AS MEDICAMENTS**

(71) Applicant: Institute Of Biotechnology Cas, V.V.I., 252 50 Vestec (CZ); Smart Brain S.r.o., 14220 Praha 4 (CZ)
(72) Inventor: Truksa, Jaroslav, 14100 Praha 4 (CZ); Werner, Lukas, 25169 Velke Popovice - Brtnice (CZ); Stursa, Jan, 14800 Praha 4 (CZ); Blazkova, Kristyna, 43963 Libesice u Zatce (CZ); Sandoval Acuna, Cristian Adrian, 14200 Praha 4 (CZ)
(74) Representative: Hartvichova, Katerina

(57) **Abstract**

The present invention claims compounds of general formula I: 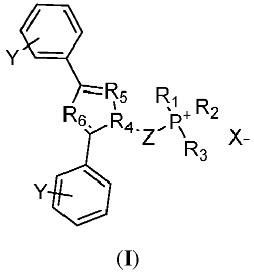 wherein the meaning of individual substituents is described in the claims.

The compounds are useful in treatment of cancers, due to their multi-modal mechanism of action.

## Description

### Field of Art

The present invention relates to novel 3,5-bis(phenyl)-1H-heteroaryl derivatives and their use as medicaments, in particular for treatment of cancers.

### Background Art

Cancers are diseases involving abnormal cell growth. The treatment of cancers is costly, and not always successful, as cancers are quite often resistant to standard chemotherapeutic treatment. There are a number of mechanisms of action through which anti-cancer medication can act. These mechanisms include anti-proliferative activity, apoptosis-inducing (or cell death-inducing) activity, iron-chelating activity, and anti-migratory activity, as well as reactive oxygen species (ROS) overproduction.

All cells require iron for their DNA synthesis, metabolism, growth and proliferation. Iron represents an indispensable micronutrient required for many enzymatic reactions such as the catalytic activity of ribonucleotide reductase for the synthesis of deoxyribonucleotides. It is also indispensable for mitochondrial respiration, due to its ability to accept and donate electrons and participate in the electron transport chain, thus leading to the generation of the electrochemical gradient across the inner mitochondrial membrane. Since cancer cells show higher demand for iron due to their proliferative nature and altered metabolic needs, an important role of iron in tumour growth and progression is expected, and marked alterations in the iron metabolism and a specific iron metabolism-related gene signature in tumour-initiating cells has been recently reported, documenting an important role of iron in cancer stem-like cells (Rychtarcikova et al. (2017) Tumor-initiating cells of breast and prostate origin show alterations in the expression of genes related to iron metabolism. Oncotarget 8, 6376-6398).

Importantly, cancer cells seem to be more sensitive to the ROS-producing compounds due to their naturally elevated basal ROS levels and almost saturated antioxidant defense mechanisms. Thus any increase in ROS is deleterious for a cancer cell that has a very limited capacity to increase its antioxidant defense, and this approach has been used for designing the anti-cancer therapy (Kim et al. (2016). ROS homeostasis and metabolism: a critical liaison for cancer therapy. Exp Mol Med 48, e269; Truksa et al. (2015). Mitochondrially targeted vitamin e succinate modulates expression of mitochondrial DNA transcripts and mitochondrial biogenesis. Antioxid Redox Signal 22, 883-900).

Moreover, since mitochondria represent a key organelle in the prodtion of heme and FeS clusters for all the proteins that require them as a cofactor (Paul and Lill (2015). Biogenesis of cytosolic and nuclear iron-sulfur proteins and their role in genome stability. Biochim Biophys Acta 1853, 1528-1539), any interference with the mitochondrial machinery should result in a decrease in metabolic activity, respiration and ability to repair DNA, leading thus to an arrest in cell cycle, inability to proliferate even if the cell is not committing apoptosis and survives.

Furthermore, a link between iron and the recycling of mitochondria via a process called mitophagy has been drawn and connected to the activation of adaptive immune system, suggesting that iron handling and mitophagy can modify the carcinogenic process (Ziegler et al. (2018). Mitophagy in Intestinal Epithelial Cells Triggers Adaptive Immunity during Tumorigenesis. Cell 174, 88-101 e116).

Recently, Novartis has introduced a new chelator, known as deferasirox (DFX, marketed as Exjade,Desirox, Defrijet, Desifer, Rasiroxpine and Jadenu) and approved by FDA in the US in 2005 (Tyagi et al. (2005). Deferasirox (ICL670; Exjade(R)), a Novel Orally Available Iron Chelator for Correction of Transfusion-Associated Iron Overload. Support Cancer Ther 2, 208-211). DFX has been shown to possess the anti-cancer activity and this has been documented *in vitro* as well as *in vivo* (Tury et al. (2018). The iron chelator deferasirox synergises with chemotherapy to treat triple-negative breast cancers. J Pathol 246, 103-114).

The aim of the present invention is to provide compounds having cytostatic effects at very low levels (nM) and cytotoxic effects at a higher concentration (µM), without showing undesirable systemic effects. The effects of the compounds should be based on multimodal mechanism of action.

### Disclosure of the Invention

The present invention provides novel compounds of general formula I, 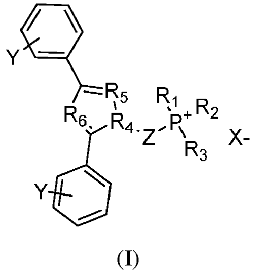 wherein
Z is a linear hydrocarbyl chain selected from alkylene, alkenylene and alkynylene, containing 2 to 20 carbon atoms, preferably 6 to 16 carbon atoms, more preferably 6 to 14 carbon atoms, even more preferably 8 to 12 carbon atoms, most preferably 10 carbon atoms, or preferably 8 to 16 or 10 to 15 carbons, wherein
   - one or more carbon atoms (typically -CH₂- groups) in the hydrocarbyl chain may optionally be replaced by one or more 5-membered or 6-membered aromatic rings or heteroaromatic rings containing the heteroatoms O, S and/or N, said aromatic or heteroaromatic rings being preferably selected from phenylene, triazolylene or pyridylene, and/or
   - one or more carbon atoms (typically -CH₂- groups) in the hydrocarbyl chain may optionally be replaced by one or more heteroatoms or heteroatom-containing moieties selected from O, S, NH, N-OH, and/or
   - the hydrocarbyl chain atoms can be unsubstituted or substituted by one or more substituents selected independently from the group comprising C1-C4 alkyl, C1-C4 alkoxy, C1-C4 alkylthio, C1-C4 acyloxy, N(H or C1-C4 alkyl)₂ wherein alkyls are the same or different, phenyl, benzyl, OH, =O, SH, =S, =N-OH, F, Cl, Br, I;
each of R1, R2, R3 is independently selected from the group comprising C1-C10 alkyl, C6-C12 aryl, C6-C12-aryl-Cl-C2-alkyl, C5-C12 heteroaryl, C3-C8 cycloalkyl, wherein each of R1, R2, R3 can optionally (and independently from others) be substituted by one or more substituents selected independently from the group comprising C1-C4 alkyl; C1-C4 alkoxy; C1-C4 alkylthio; N(H or C1-C4 alkyl)₂ wherein the alkyls are the same or different; OH; =O; SH; =S; =N-OH; F; Cl; Br; I;
each of R4, R5 and R6 is selected from CH and N, whereas at least two of R4, R5 and R6 are N;
X⁻ is a pharmaceutically acceptable anion;
Y is independently on each occurrence selected from the group comprising OH, SH, NH₂, F, Cl, Br, I and H.

The definition of formula I is intended to include any salts, solvates, isomers and mixtures of isomers.

X⁻ is typically selected from anions of inorganic or organic acids, particularly suitable are Cl⁻, Br⁻, I⁻, carbonate, bicarbonate, nitrate, sulphate, phosphate, acetate, ascorbate, aspartate, benzenesulfonate, benzoate, besylate, bitartrate, camsylate, citrate, decanoate, edetate, esylate, formiate, fumarate, gluceptate, gluconate, glutamate, glycolate, hexanoate, hydroxynaphthoate, isethionate, lactate, lactobionate, malate, maleate, mandelate, mesylate, methylsulfate, mucate, napsylate, octanoate, oleate, oxalate, pamoate, pantothenate, polygalacturonate, propionate, salicylate, stearate, succinate, tartrate, teoclate, tosylate, but anions of any pharmaceutically acceptable acids can be used.

In a preferred embodiment, R6 is N, and at least one or R4 and R5 is also N.

In a preferred embodiment, Z is selected from an alkylene; an alkylene wherein one carbon atom is replaced by a 5-membered or 6-membered aromatic ring or heteroaromatic ring containing the heteroatoms O, S and/or N; an alkylene wherein one carbon atom is replaced by phenylene, triazolylene or pyridylene; an alkylene wherein one carbon atom is replaced by NH; an alkylene wherein one carbon atom is replaced by phenylene, triazolylene or pyridylene, one carbon atom is replaced by NH and one carbon atom is substituted by =O, thus preferably forming the structure - (hetero)aryl-C(=O)-NH-. In this preferred embodiment, Z may optionally be further substituted.

In a preferred embodiment, Y is H, OH, F or SH. Most preferably, Y is OH.

Furthermore, the present invention provides the compounds of formula I for use as medicaments, in particular for use for treatment of cancers. The cancers may include, for example, breast, ovarian, prostate, GIT, hepatic, colorectal, pancreatic, mesothelioma, lung cancers and leukaemias.

The compounds of the present invention act by several independent mechanisms which makes them suitable for treatment of various types of cancers, including resistant cancers. The mechanisms of action of the compounds of formula I include anti-proliferative activity, apoptosis-inducing (or cell death-inducing) activity, metal-chelating activity, in particular iron-chelating activity, and anti-migratory activity. These activities are selective to malignant cells. Resistant cancers are typically resistant to medicaments acting through a certain mechanism of action. The compounds of the present invention having multiple modes of action may then overcome the resistance by acting through a different mode of action to which the cancer cells are sensitive.

Compared to the structurally partially similar substance, deferasirox, the representative compounds of the present invention show an IC₅₀ towards malignant cells which is 3-4 orders of magnitude lower than the IC₅₀ of deferasirox. Compared to another structurally similar compound, a triphenylphosphoniumdecyl derivative of tamoxifen (mitoTAX, WO2014/173374), the representative compounds of the present invention show an IC₅₀ towards malignant cells which is 1-2 orders of magnitude lower than the IC₅₀ of that of mitoTAX.

Object of the present invention is also a pharmaceutical composition containing at least one compound of general formula I and at least one pharmaceutically acceptable excipient, such as a carrier, a solvent, a filler, a binder, a disintegrant, a coating, a glidant, a lubricant, a preservative, a flavor, a colorant.

Additionally, the present invention includes a pharmaceutically acceptable composition comprising at least one compound of formula I and a metal. The metal is preferably selected from transition metals (B-groups of the periodic table, lanthanides, actinides) and metals of IIIA and IVA groups of the periodic table. The metal may be a radionuclide or a metal suitable for use as a diagnostic or therapeutic agent, for example for radiation therapy, biosensing, bioimaging, drug delivery, gene delivery, photodynamic therapy (in particular metals such as lanthane, cerium, praseodyme, neodyme, promethium, samarium, europium, gadolinium, terbium, dysprosium, holmium, erbium, thulium, ytterbium, lutecium, iron, gallium, copper). At least part of the total amount of the metal and at least part of the total amount of compounds of formula I present in the preparation may form a chelate complex.

Object of the present invention are thus the compounds of general formula I or the pharmaceutical preparation comprising at least one compound of formula I and a metal, preferably gallium, for use as medicaments, in particular for use in a method of treatment of cancer.

A further object of the present invention is use of the compounds of general formula I or of the pharmaceutical preparation comprising at least one compound of formula I and a metal, preferably gallium, for preparation of a medicament for the treatment of cancer.

Further, object of the present invention is a method of treatment of mammals, including human, in which one or more compounds of general formula I is administered to a subject suffering from cancer.

Object of the present invention is also a pharmaceutical composition comprising at least one compound of formula I and a metal, preferably gallium, for use as a contrast agent, used for diagnostic in particular for use in a method of *in vivo* visualisation of cancer. Such preparation can be visualized by, e.g., positron emission tomography (PET).

### Examples of carrying out the Invention

### Example 1

### (10-(4-(3,5-bis(2-hydroxyphenyl)-1H-1,2,4-triazol-1-yl)benzamido)decyl) triphenylphosphonium chloride (Compound 1)

Deferasirox (114 mg, 0.31 mmol) with 10-aminodecylphosphonium chloride hydrochloride (150 mg, 0.31 mmol), N-(3-dimethylaminopropyl)-N'-ethylcarbodiimide hydrochloride (EDC) (88 mg, 0.46 mmol) and N,N-diisopropylethylamine (DIPEA) (0.32 ml, 1.86 mmol) were dissolved in dimethylformamide (DMF) (3 ml) and reaction was stirred for 72 hours. Solvents were evaporated, mixture was dissolved in methanol (MeOH) and extracted with citric acid (5 %), brine and dichloromethane (DCM). Pure product of the formula 1 (25 mg, 10 %), isolated as two rotamers was obtained by column chromatography (2% MeOH/CHCl₃) as light yellow foam. 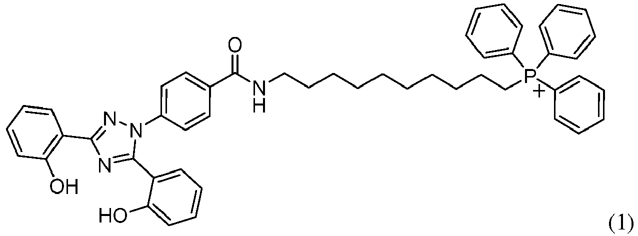 ¹H NMR (500 MHz, Chloroform-d) δ 10.96 (bs, OH, 1H), 10.16 (bs, OH, 1H), 8.40 (bs, NH, 1H), 8.12 (t, J = 7.9 Hz, 2H), 7.79 - 7.68 (m, 6H), 7.65 (s, 7H), 7.42 (d, J = 6.9 Hz, 2H), 7.33 (t, J = 7.9 Hz, 1H), 7.25 (dd, J = 26.3, 7.7 Hz, 2H), 7.11 (d, J = 7.7 Hz, 1H), 7.00 (m, 2H), 6.74 (t, J = 7.4 Hz, 1H), 3.46 (m, 4H), 1.79 - 1.48 (m, 6H), 1.26 (m, 10H).
¹³C NMR (126 MHz, Chloroform-d) δ 166.48, 159.61, 157.18, 156.65, 152.14, 139.81, 135.84, 135.10, 133.52 (d, J = 9.9 Hz), 132.60, 131.39, 130.52 (d, J = 12.4 Hz), 129.14, 128.79, 127.31, 124.61,119.65,19.24,118.15 (d, J = 85.74 Hz), 117.95, 117.05, 113.65, 111.91,40.17,30.19,30.06, 29.69, 29.00, 28.59, 28.50, 28.46, 26.61, 22.83, 22.51, 22.47.
HRMS calculated for C49H50O3N4P⁺: 773.36150, found: 773.36128.

### Example 2

### (10-(3,5-bis(2-hydroxyphenyl)-1H-1,2,4-triazol-1-yl)decyl)triphenylphosphonium bromide (Compound 2)

(10-bromodecyl)triphenylphosphonium bromide (750 mg, 1.334 mmol) was heated with hydrazine (6 ml) for 2 hours. Reaction mixture was cooled to laboratory temperature, diluted with H₂O (50 ml) and extracted 2 x with DCM and dried over MgSO₄. Crude material was concentrated, dried under vacuum, diluted with ethanol (6 ml) and 2-(2-hydroxyphenyl)-4H-benzo[e][1,3]oxazin-4-one (377 mg, 1.576 mmol) was added and reaction mixture was refluxed for 1.5 hours. Reaction mixture was concentrated under vacuum and purified by column chromatography (toluene: methanol, gradient 100:5 - 100:15) to yield product (330 mg, 30 %) of formula 2 as light yellow foam. 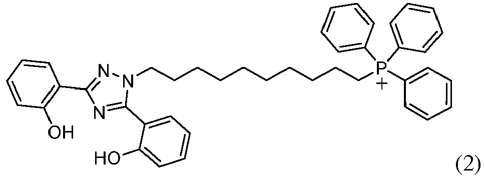 ¹H NMR (600 MHz, Chloroform-*d*) δ 10.55 (s, 1H), 8.06 (dd, *J* = 7.8, 1.7 Hz, 1H), 7.83 - 7.70 (m, 9H), 7.65 (td, *J* = 8.0, 3.4 Hz, 6H), 7.48 - 7.43 (m, 2H), 7.35 (ddd, *J* = 8.5, 7.2, 1.6 Hz, 1H), 7.31 - 7.25 (m, 2H), 7.22 - 7.16 (m, 1H), 6.99 (ddd, *J* = 7.3, 6.5, 1.0 Hz, 2H), 6.95 (td, *J* = 7.5, 1.1 Hz, 1H), 4.28 (t, *J* = 7.4 Hz, 2H), 3.64 (td, *J* = 12.6, 5.1 Hz, 2H), 1.96 (p, *J* = 7.5 Hz, 2H), 1.61 - 1.50 (m, 4H), 1.35 - 1.28 (m, 2H), 1.28 - 1.10 (m, 8H).
¹³C NMR (151 MHz, Chloroform-*d*) δ 159.10, 156.71, 156.55, 152.15, 134.96 (d, *J* = 3.0 Hz), 133.58 (d, *J* = 10.7 Hz), 132.20, 130.85, 130.43 (d, *J* = 12.5 Hz), 128.23, 126.93, 119.48, 119.40, 118.27 (d, *J* = 85.8 Hz), 117.97, 116.90, 114.28, 112.61, 50.23, 30.12 (d, *J* = 15.8 Hz), 29.28, 28.73 (overlap), 28.68 (d, *J* = 1.1 Hz), 26.18, 22.80, 22.51, 22.48 (overlap), 22.47.
HRMS calculated for C42H45O2N3P⁺: 654.32439, found: 654.32443.

### Example 3

### 2,2'-(1-(9-hydroxynonyl)-1H-1,2,4-triazole-3,5-diyl)diphenol (Compound 3)

10-Bromo-1-nonanol (117 mg, 0.524 mmol) was mixed with hydrazine (2 ml) and stirred vigorously at 80°C for 2 hours. The reaction was cooled to laboratory temperature, water (20 ml) was added and the mixture was extracted 3 times with DCM (15 ml). Crude material was concentrated under vacuum, diluted with ethanol (4 ml) and 2-(2-hydroxyphenyl)-4H-benzo[e][1,3]oxazin-4-one (187 mg, 0.782 mmol) was added. The reaction mixture was heated at 80°C for 3 hours Crude product was concentrated under vacuum and purified by column chromatography (chloroform: methanol, gradient 100:0 - 100:1) to yield product (70 mg, 24 %) of the formula 3 as a light yellow foam. 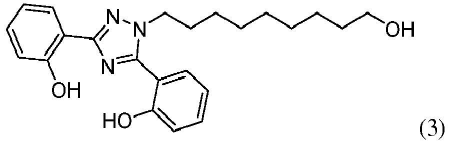 ¹H NMR (600 MHz, Chloroform-*d*) δ 11.18 (s, 1H), 9.89 (s, 1H), 8.10 (dd, *J* = 7.8, 1.7 Hz, 1H), 7.57 (dd, *J* = 7.9, 1.6 Hz, 1H), 7.46 (ddd, *J* = 8.3, 7.3, 1.6 Hz, 1H), 7.37 (ddd, J= 8.3, 7.2, 1.7 Hz, 1H), 7.19 (dd, *J* = 8.4, 1.2 Hz, 1H), 7.09 (dd, *J* = 8.3, 1.1 Hz, 1H), 7.04 (qd, *J* = 7.9, 1.1 Hz, 2H), 4.47 (t, *J* = 7.4 Hz, 2H), 3.66 (t, *J* = 6.6 Hz, 2H), 2.11 - 1.99 (m, 2H), 1.61 - 1.54 (m, 2H), 1.47 - 1.41 (m, 2H), 1.40 - 1.28 (m, 8H).
¹³C NMR (151 MHz, Chloroform-*d*) δ 158.47, 157.54, 156.30, 151.80, 132.53, 131.33, 127.29, 126.67, 119.76, 119.46, 118.33, 116.94, 113.72, 110.93, 62.97, 50.81, 32.65, 29.51, 29.24, 29.16, 28.87, 26.38, 25.60.
HRMS calculated for C23H30O3N3⁺: 396.22817, found: 396.22771.

### Example 4

### (10-(3,5-bis(2-hydroxyphenyl)-1H-1,2,4-triazol-1-yl)decyl)tricyclohexylphosphonium bromide

Compound 3 (40 mg, 0.101 mmol) was dissolved in DCM (1 ml), cooled to 0-4 °C and triphenylphosphine dibromide (171mg, 0.404 mmol) was added in one portion. Reaction mixture was allowed to warm up to laboratory temperature and DIPEA (0.2 ml) was added. The mixture was stirred overnight at room temperature. Reaction was quenched with H₂O (0.2 ml) and DMF (2 ml) was added. H₂O and DIPEA were partially removed under vacuum and tricyclohexyl phosphine (1 g, 3.566 mmol) was added. Reaction mixture was heated 1 hour under vacuum at 90 °C and then heating continued under inert atmosphere overnight. Reaction mixture was cooled to laboratory temperature and cooled mixture was added dropwise into cold (4 °C) diethylether (200 ml). White precipitate was formed, solvent decanted off and crude product was purified with column chromatography to yield product (30 mg, 40 %) of the formula 4. 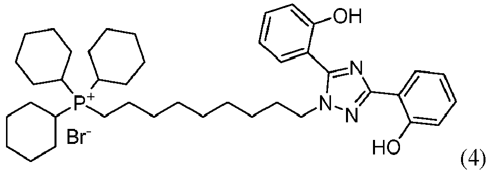 ¹H NMR (600 MHz, Chloroform-d) δ 10.80 (s, 1H), 8.09 (dd, J = 7.8, 1.7 Hz, 1H), 7.58 - 7.52 (m, 1H), 7.44 (dd, J = 7.7, 1.7 Hz, 1H), 7.42 - 7.32 (m, 2H), 7.30 (ddd, J = 8.7, 7.2, 1.7 Hz, 1H), 7.02 (dd, J = 8.3, 1.1 Hz, 1H), 7.00 - 6.97 (m, 1H), 6.96 (dd, J = 7.5, 1.1 Hz, 1H), 4.26 (t, J = 7.2 Hz, 2H), 2.55 - 2.44 (m, 3H), 2.39 (dt, J = 19.3, 6.4 Hz, 2H), 2.02 - 1.90 (m, 8H), 1.90 - 1.83 (m, 6H), 1.81 - 1.69 (m, 4H), 1.57 - 1.16 (m, 27H).
¹³C NMR (151 MHz, Chloroform-d) δ 159.32, 156.63, 156.55, 152.31, 132.12, 130.78, 128.52, 126.82, 119.46, 119.34, 117.85, 116.91, 114.37, 113.01, 49.96, 30.68 (d, J = 13.8 Hz), 29.90 (d, J = 40.4 Hz), 29.07, 28.36 (d, J = 17.2 Hz), 27.95, 27.17, 27.14, 26.50, 26.42, 25.76, 25.40, 22.50 (d, J = 5.4 Hz), 15.73 (d, J = 42.6 Hz).
HRMS calculated for C41H61O2N3P⁺: 658.44959, found: 658.44945.

### Example 5

### 4-(2-(tert-butoxycarbonyl)hydrazineyl)benzoic acid (Compound 5)

4-hydrazinobenzoic acid (500 mg, 3.286 mmol) and Boc anhydride (720 mg, 3.299 mmol, Boc = di(tert-butyl)dicarbonate) were dissolved in DMF (4 ml), DIPEA (1 ml) was added and reaction mixture was stirred for 1 hour. Reaction mixture was concentrated and dried under vacuum. Crude material was purified on column chromatography (chloroform: methanol, 100:10) to get product (690 mg, 79 %) of the formula 5 in the form of yellowish foam. 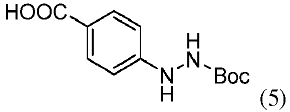 ¹H NMR (600 MHz, Methanol-d4) δ 7.88 - 7.76 (m, 2H), 6.78 - 6.65 (m, 2H), 1.46 (s, 6H).
¹³C NMR (151 MHz, Methanol-d4) δ 170.37, 158.67, 154.93, 132.45, 121.78, 112.01, 81.54, 28.63. HRMS calculated for C12H17O4N2⁺: 253.11828, found: 253.11779.

### Example 6

### (10-ammoniodecyl)triphenylphosphonium chloride hydrochloride (Compound 6)

(10-bromodecyl)triphenylphosphonium bromid (9.26 g, 16.448 mmol) was dissolved in 7M solution of ammonia in methanol (60 ml) and the reaction mixture was stirred at 50 °C. After 6 h, additional ammonia in methanol (40 ml) was added and the reaction mixture was heated for additional 24 h at 50°C. The reaction mixture was concentrated under reduced pressure, and crude product was purified using column chromatography on silicagel (200 ml) (chloroform/methanol gradient 0-10% of methanol). The product was acidified with HCl (36%, 5 ml) and filtered through Dowex 1x8 in chloride form (50 g) to obtain desired (10-aminodecyl)triphenylphosphonium chloride hydrochloride (5.135 g, 63%) of the formula 6. 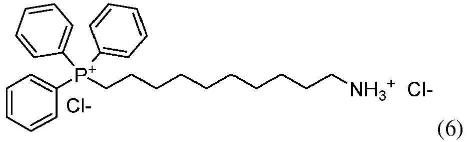 ¹H NMR (500 MHz, Methanol-d4) δ 7.92 - 7.85 (m, 3H), 7.85 - 7.68 (m, 12H), 3.46 - 3.37 (m, 2H), 2.90 (t, J = 7.5 Hz, 2H), 1.72 - 1.60 (m, 4H), 1.56 (p, J = 7.5 Hz, 2H), 1.43 -1.20 (m, 12H)
¹³C NMR (126 MHz, Methanol-d4) δ 136.23 (d, J = 3.0 Hz), 134.79 (d, J = 9.9 Hz), 131.51 (d, J = 12.5 Hz), 120.00 (d, J = 86.3 Hz), 40.76, 31.59 (d, J = 16.2 5 Hz), 30.29 (2C), 30.12, 29.89, 28.53, 27.42, 23.57 (d, J = 4.4 Hz), 22.68 (d, J = 51.1 Hz).
IR (KBr pellet): v = 3051, 3007, 2927, 2854, 2006, 1825, 1601, 1587, 1485, 1465, 1438, 1402, 1337, 1318, 1189, 1161, 1113, 996, 751, 723, 691.
HRMS calculated for C28H37NP⁺: 418.26581, found: 418.26567.

### Example 7

### (10-(2,4-bis(2-hydroxyphenyl)-1H-imidazol-1-yl)decyl)triphenylphosphonium chloride (Compound 7)

Compound 5 (100 mg, 0.397 mmol), Compound 6 (389 mg, 0.793 mmol), O-(benzotriazol-1-yl)-N,N,N',N'-tetramethyluronium tetrafluoroborate (TBTU) (255 mg, 0.794 mmol) and triethylamine (Et₃N) (0.55 ml, 3.9 mmol) were dissolved in DMF (1 ml) and reaction mixture was stirred for 2 h. The reaction mixture was added dropwise into ice cooled diethylether (Et₂O) (50 ml) and oily precipitate formed on the walls of flask upon stirring in ice-bath. Solvent was decanted off and precipitate was dissolved in methanol (3mL) and diluted with half saturated solution of NH₄Cl (10 mL). Resulting milky solution was extracted DCM (3 x 15 mL). Combined organic layer was dried over MgSO₄ and concentrated under vacuum. The identical extraction procedure (3 mL of methanol, 10 mL NH₄Cl, 3 x 15 mL DCM) was repeated two more times. The crude product was purified by column chromatography (chloroform/methanol gradient 0-10% of methanol) to afford product of the formula 7 (170 mg, 62 %) as a yellowish foam. 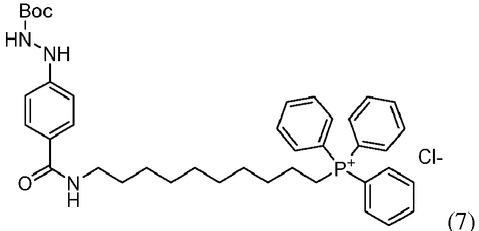 ¹H NMR (600 MHz, Methanol-d4) δ 7.93 - 7.74 (m, 15H), 7.69 (d, J = 8.7 Hz, 2H), 6.81 - 6.75 (m, 2H), 3.44 - 3.37 (m, 2H), 1.67 (h, J = 8.2 Hz, 2H), 1.59 (q, J = 6.9 Hz, 2H), 1.55 (t, J = 7.5 Hz, 2H), 1.51 (s, 6H), 1.33 (d, J = 24.7 Hz, 12H).
¹³C NMR (151 MHz, Methanol-d4) δ 168.69, 157.33, 152.31, 134.85, 133.39 (d, J = 10.0 Hz), 130.09 (d, J = 12.6 Hz), 128.20, 124.42, 118.88, 118.31, 110.92, 39.39, 30.11 (d, J = 16.1 Hz), 29.14, 28.93, 28.81 (d, J = 7.1 Hz), 28.34, 27.23, 26.54, 22.13 - 21.97 (m), 21.32, 20.99.
HRMS calculated for C40H51O3N3P⁺: 652.36626, found: 652.36596.

### Example 8

### 2-(3,5-bis(2-hydroxyphenyl)-1H-1,2,4-triazol-1-yl)benzoic acid (Compound 8)

2-hydrazinobenzoic acid hydrochloride (200 mg, 1.060 mmol) and 2-(2-hydroxyphenyl)-4H-benzo[e][1,3]oxazin-4-one (231 mg, 0.966 mmol) were dissolved in EtOH (10 ml) and the reaction mixture was heated to 70 °C. Et₃N (0.28 ml, 2.001 mmol) was added dropwise. The reaction mixture was stirred for 4 hours and then water (5 ml) was added. EtOH was evaporated and 6 M HCl (3 ml) was added. Crude product precipitated as light yellow solid. Pure product of the formula 8 was obtained with column chromatography on silicagel (30 ml) (chloroform/methanol, gradient 0-5% of methanol) as light yellow foam (333 mg, 92%). 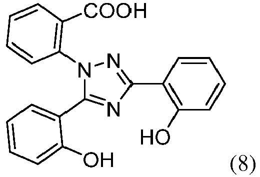 ¹H NMR (600 MHz, DMSO-d6) δ 8.00 (dd, J = 7.8, 1.7 Hz, 1H), 7.89 (dd, J = 7.7, 1.6 Hz, 1H), 7.73 (td, J = 7.7, 1.6 Hz, 1H), 7.66 - 7.60 (m, 2H), 7.36 (ddd, J = 8.3, 7.2, 1.7 Hz, 1H), 7.29 (ddd, J = 8.3, 7.3, 1.8 Hz, 1H), 7.20 (dd, J = 7.7, 1.7 Hz, 1H), 7.02 (dd, J = 8.3, 1.1 Hz, 1H), 6.99 (td, J = 7.5, 1.2 Hz, 1H), 6.87 (dd, J = 8.3, 1.0 Hz, 1H), 6.82 (td, J = 7.6, 1.1 Hz, 1H).
¹³C NMR (151 MHz, DMSO-d6) δ 166.28, 159.63, 156.72, 156.20, 153.33, 136.99, 132.57, 131.63, 131.04 (d, J = 23.5 Hz), 130.46, 128.77, 127.10, 120.03, 119.40, 117.41, 116.71, 114.37, 113.95. HRMS calculated for C21H16O4N3⁺: 374.11353, found: 374.11324.

### Example 9

### (10-(2-(3,5-bis(2-hydroxyphenyl)-1H-1,2,4-triazol-1-yl)benzamido)decyl) triphenylphosphonium chloride (Compound 9)

Compound 8 (120 mg, 0.322 mmol), compound 6 (320 mg, 0.642 mmol), EDC (123 mg, 0.642 mmol) and Et₃N (0.22 ml, 1.6 mmol) were dissolved in DMF (5 ml) and reaction was stirred for 2 hours. Crude product was precipitated by dropwise addition of reaction mixture into in ice cooled Et₂O (150 mL). The crude product was dissolved in MeOH (3 mL) and citric acid (5 %, 3 mL) and half saturated aqueous NH₄Cl (10 mL) and extracted with DCM (3 x 30 mL). Pure product (34 mg, 13 %) of the formula 9, was obtained by column chromatography (toluene/methanol, gradient 100:5 - 100:20) as light yellow foam. 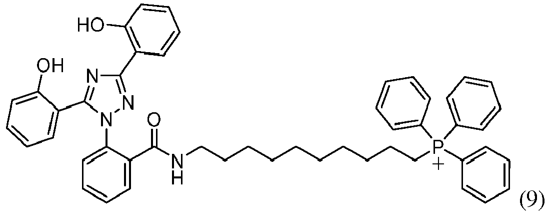 ¹H NMR (600 MHz, Chloroform-d) δ 10.55 (s, 1H), 8.14 (dd, J = 7.8, 1.7 Hz, 1H), 7.80 (ddd, J = 19.6, 14.1, 8.1 Hz, 9H), 7.69 (td, J = 7.7, 3.3 Hz, 6H), 7.53 - 7.48 (m, 1H), 7.46 - 7.29 (m, 7H), 7.24 (d, J = 8.4 Hz, 1H), 7.19 - 7.11 (m, 1H), 7.09 (dd, J = 7.9, 1.1 Hz, 1H), 7.01 (dd, J = 8.2, 1.1 Hz, 1H), 6.94 (ddd, J = 8.1, 7.3, 1.1 Hz, 1H), 6.81 (t, J = 7.5 Hz, 1H), 3.60 (m, 2H), 3.19 (q, J = 6.6 Hz, 2H), 1.90 - 1.61 (m, 2H), 1.60 - 1.48 (m, 2H), 1.46 - 1.06 (m, 12H).
¹³C NMR (151 MHz, Chloroform-d) δ 166.54, 160.31 , 156.76 , 156.47 , 153.04 , 135.03 (d, J = 2.9 Hz), 134.96 , 134.22 , 133.51 (d, J = 9.9 Hz), 132.01 , 131.06 , 130.43 (d, J = 12.5 Hz), 128.96 , 128.56, 127.36, 126.26, 119.43, 119.11 , 118.30 (d, J = 85.8 Hz), 117.69, 116.94, 113.88, 112.87 , 39.77 , 29.69 , 29.52 (d, J = 16.0 Hz), 28.33 , 28.04 , 28.03 , 27.92 , 27.66 , 25.93 , 22.15 (d, J = 33.7 Hz).
HRMS calculated for C49H50O3N4P⁺: 773.36150, found: 773.36134.

### Example 10

### (10-(2,4-bis(2-hydroxyphenyl)-1H-imidazol-1-yl)decyl)triphenylphosphonium chloride (Compound 10)

To a solution of 2-Bromo-2'-hydroxyacetophenone (108 mg, 0.5 mmol) in DMF (2 mL) compound 6 (138 mg; 0.303 mmol)) was added in one portion. Mixture was heated to 90 °C for 3 hours. Then salicylaldehyde (122 mg; 1.0 mmol) and ammonium acetate (77 mg; 1.0 mol) was added. Mixture was heated overnight to (90 °C). TLC analysis (CHCl₃/MeOH/CH₃COOH 100:10:1) indicated formation of complex reaction mixture where product can be visualized by 5 % solution of FeCl₃. Reaction mixture was then cooled to room temperature and diluted with diethylether (20 mL). Brown precipitate formed on the walls of reaction vessel. Solvent was decanted off and precipitate was dissolved in methanol (3 ml). Upon addition of half saturated solution of ammonium chloride (15 ml) into the methanolic solution of crude product, milky emulsion formed. Emulsion was extracted by dichloromethane (3 x 50 ml) and combined organic layer was dried over MgSO₄. Organic layer was then concentrated under vacuum and subjected to column chromatography (10 ml of silica, Toluene/methanol 100:5 - 100:20). Combined fractions of product of the formula 10 afforded (23 mg, 11 %) in the form of yellow oil. 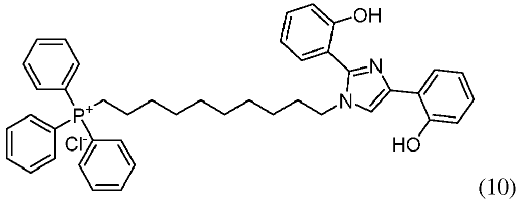 ¹H NMR (600 MHz, Chloroform-d) δ 11.67 (s, 1H), 8.12 (dd, J = 7.7, 1.6 Hz, 1H), 7.83 - 7.70 (m, 10H), 7.55 (td, J = 8.2, 3.6 Hz, 6H), 7.54 - 7.39 (m, 2H), 7.30 (ddd, J = 8.4, 7.3, 1.8 Hz, 1H), 7.30 - 7.20 (m, 2H), 7.20 - 7.16 (m, 1H), 6.83 (ddd, J = 7.1, 6.4, ≈1.0 Hz, 2H), 6.85 (td, J = 7.4, ≈1.1 Hz, 1H), 4.15 (t, J = 7.4 Hz, 2H), 3.60 (td, J = 11.9, 5.5 Hz, 2H), 1.99 (p, J = 7.5 Hz, 2H), 1.65 - 1.45 (m, 4H), 1.38 - 1.27 (m, 2H), 1.25 - 1.10 (m, 8H).
¹³C NMR (151 MHz, Chloroform-d) δ 157.20, 154.88, 153.75, 142.61, 135.72 (d, J = 3.1 Hz), 132.33 (d, J = 11.5 Hz), 133.54, 131.62, 129.12 (d, J = 12.5 Hz), 127.47, 126.8, 119.48, 117.40, 117.16 (d, J = 87.1 Hz), 116.90, 116.22, 114.28, 113.66, 48.49, 30.69 (d, J = 16.3 Hz), overlap (28.12, 28.11, 28.05), 26.00, 23.00, 22.38, 22.28 (overlap), 21.12.

### Example 11

### (10-(4-(3,5-bis(2-hydroxyphenyl)-1H-1,2,4-triazol-1-yl)benzamido)decyl) triphenylphosphonium (Compound 1)

Compound 6 (50 mg, 0.073 mmol) was dissolved in dry DCM (1 ml) and TFA (1 ml) was added dropwise. Reaction mixture was stirred for 2.5 hours, then the solvent was evaporated and the residue was dissolved in ethanol (2 ml). 2-(2-hydroxyphenyl)-4H-benzo[e][1,3]oxazin-4-one (35 mg, 0.146 mmol) was slowly added to the reaction mixture and Et₃N (0.02 ml, 0.15 mmol) was added dropwise. The reaction mixture was heated to 70 °C and stirred for 2 hours. Solvent was evaporated. Mixture was dissolved in MeOH and 3x extracted with saturated aqueous NH₄Cl and DCM. Pure product was obtained (34 mg, 57 %) by column chromatography on silicagel (12 ml) (toluene/methanol, gradient 0-10% of methanol) as light yellow foam.

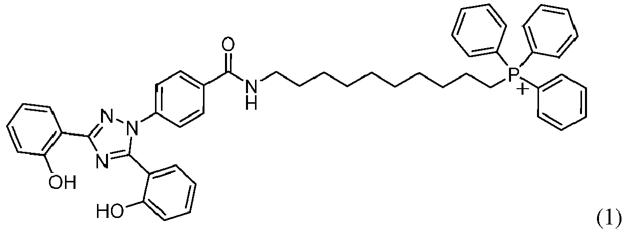

### Example 12

Compound 1 and a comparative compound deferasirox (DFX) were tested in their efficacy to kill/ suppress proliferation of malignant breast cancer MCF7 cells representing estrogen-dependent type of breast cancer. Briefly, 10.000 cells per well were seeded in a 96-well plate on one day and the next day the tested compounds were added and incubated with the cells for 48 hrs. The cells were then fixed with 4% paraformaldehyde, stained with 0.05% crystal violet, washed with PBS, and solubilized in 1% SDS. The absorbance of the plate at 595 nm was then measured, quantifying the number of viable cells. The results show that the compound 1 was very effective in comparison with deferasirox and showed a cytostatic effect that lead to suppression of proliferation to 50% (IC₅₀) at a concentration as low as 8 nM compared to 10 µM of deferasirox, thus showing the same effect at a concentration lower by four orders of magnitude. The comparison is shown in Table 1.

**Table 1: The effect of DFX and compound 1 on cellular viability in malignant MCF7 cells after 48hrs of incubation as measured with crystal violet assay that combines cytostatic as well as cytotoxic effect. Data represent mean percentages of viable cells compared to non-treated controls while numbers in brackets represent standard deviations.**

| Concentration | Compound 1 | DFX |
|---|---|---|
| 0 µM | 100.00 (8.82) | 100.00 (8.82) |
| 0.030 µM | 33.73 (8.88) | - |
| 0.100 µM | 23.65 (7.48) | - |
| 0.300 µM | 14.75 (3.80) | - |
| 1 µM | 7.28 (1.15) | 103.83 (11.68) |
| 3 µM | 5.20 (2.53) | 76.03 (12.85) |
| 10 µM | 2.31 (2.67) | 56.52 (1.43) |
| 30 µM | 0.69 (0.23) | 23.89 (7.38) |
| IC₅₀ (µM) | 0.008 (0.003) | 10.90 (1.48) |

### Example 13

Compound 1 and the comparative compound deferasirox were tested in their efficacy to kill/suppress proliferation of malignant MDA-MB-231 cancer cells representing hard to treat triple negative breast cancer. The procedure is identical to that in example 12. The results show that the compound 1 was very effective in comparison with deferasirox and shows IC₅₀ value of 131 nM compared to 70 µM for deferasirox, thus showing a difference of almost three orders of magnitude. The comparison is shown in Table 2.

**Table 2: The effect of DFX and compound 1 on cellular viability in malignant MDA-MB-231 cells after 48hrs of incubation as measured with crystal violet assay that combines cytostatic as well as cytotoxic effect. Data represent mean percentages of viable cells compared to non-treated controls while numbers in brackets represent standard deviations.**

| Concentration | Compound 1 | DFX |
|---|---|---|
| 0 µM | 100.00 (5.82) | 100.00 (6.60) |
| 0.030 µM | 71.10 (7.60) | - |
| 0.100 µM | 52.47 (8.87) | - |
| 0.300 µM | 37.42 (5.70) | - |
| 1 µM | 27.81 (2.09) | 109.15 (14.65) |
| 3 µM | 18.23 (5.89) | 90.81 (12.17) |
| 10 µM | 0.29 (1.46) | 70.38 (2.35) |
| 30 µM | 0.51 (0.38) | 62.07 (11.57) |
| 100 µM | - | 55.70 (15.68) |
| 300 µM | - | 14.09 (4.57) |
| IC₅₀ (µM) | 0.131 (0.019) | 70.02 (15.26) |

### Example 14

Compounds 1, 2 and the comparative compound deferasirox were tested in their efficacy to suppress proliferation/kill several malignant cell lines of breast, pancreatic and ovarian origin (MCF7, MDA-MB-231, BxPC3 and OVCAR3). The procedure is identical to that in example 12. The results show that the selectivity is increased significantly as documented in in Table 3.

**Table 3: The effect of DFX and compounds 1 and 2 on cellular viability in non-malignant fibroblasts (HFP1) and several malignant cell lines (MCF7, MDA-MB-231, BxPC3 and OVCAR3) after 48hrs of incubation as measured with crystal violet assay that combines cytostatic as well as cytotoxic effect. Data represent mean percentages of viable cells compared to non-treated controls while numbers in brackets represent standard deviations.**

| IC₅₀ (µM) | MCF7 | T47D | MDA-MB-231 | BxPC3 | OVCAR3 | HFP1 |
|---|---|---|---|---|---|---|
| DFX | 10.90 (1.48) | 60.81 (8.92) | 70.02 (15.26) | 22.89 (8.06) | 19.18 (3.78) | 37.92 (8.37) |
| Compound 1 | 0.008 (0.003) | 0.031 (0.013) | 0.130 (0.019) | 0.150 (0.055) | 0.154 (0.042) | 0.532 (0.195) |
| Compound 2 | 0.175 (0.020) | | 0.464 (0.068) | | | |

Compound 1 shows the ability to suppress the growth of all tested cancer cells as documented in Table 3 depicting crystal violet staining performed as in example 12 (measuring the combined anti proliferative and cell death-inducing effect) of cells treated with DFX or compound 1 or 2. Importantly, the *in vitro* IC₅₀ values of newly synthesized compound 1 are almost three orders of magnitude lower as compared to the parental DFX and one to two orders of magnitude lower than other mitochondrially targeted chelators, suggesting that it is a highly active compound (Table 1, 2), reaching efficacy that is higher compared to what could be expected by mitochondrial targeting of DFX based on enhancing the effect of parental drug by two orders of magnitude as seen in other mitochondrially targeted derivatives (e.g., mitoTAX, WO2014/173374). Furthermore, compound 2 shows an IC₅₀ values in the range of 100 nM, which are higher than for compound 1, but still very potent.

### Example 15

Compound 1 shows very high efficacy against cancer cell lines in the 10-100 nM range, as seen in previous tables. This effect seems to be based mainly on the cytostatic effect of compound 1. To define the ability to kill cells, cell death-inducing potency (cytotoxic efficacy) of DFX and compound 1 have been tested *via* annexin V/PI staining commonly used as a measure of apoptotic/necrotic cell death. Briefly, cells were seeded in 12-well plate at 100,000 per well, incubated with the selected compounds for 48 hours, then floating cells were spun, adherent cells trypsinized and spun as well followed by the staining with AnnexinV-FITC and PI probes. Cells were then washed with PBS and measured via Fluorescence Activated Sorter. Cells exhibiting AnnexinV and/or PI positivity are considered as dead cells. As shown in Table 4, compound 1 exhibits a markedly enhanced capacity to induce cell death as compared to DFX in all malignant breast cancer cells. Importantly, non-malignant HFP1 cells were significantly less sensitive to the effect of compound 1 and toxic effect could be detected only in concentrations higher than 5 µM where all tested cancer cells exhibited profound proportion of dead cells (Table 4).

The compounds of the present invention were tested for their biological effects and compared with the known compound - deferasirox. The most active compounds of the present invention killed cancer cells with effects higher by almost 3 orders of magnitude than those of deferasirox.This is unprecedented and very unexpected. Compound 2 shows an IC₅₀ values in the range of 100 nM and a significantly higher cytotoxic activity suggesting that other compound of similar chemical group show similar effect and the invention is not restricted to deferasirox derivatives only.

An important finding is that the compounds of the present invention do show less toxic effects on non-malignant cells and are well tolerated in vivo, hence, they are more selective in killing the cancer cells.

**Table 4: The effect of compound 1, compound 2 and DFX on cell death induction in malignant (MCF7, BxPC3, OVCAR, MDA-MB-231) and non malignant cells (HFP1, MRC5) as measured with AnnexinV/PI assay after 48 hrs. Data represent mean percentage of dead cells while numbers in brackets represent standard deviations.**

| Concentration | MCF7 | BxPC3 | OVCAR | MDA-MB-231 | HFP1 | MRC5 |
|---|---|---|---|---|---|---|
| 0 µM | 5.4 (2.3) | 4.7 (1.6) | 8.7 (2.3) | 6.7 (1.6) | 7.6 (1.8) | 8.4 (2.6) |
| DFX 500 µM | 85.2 (3.3) | 45.8 (21.3) | 59.7 (4.6) | - | 35.6 (1.5) | 43.7 (7.7) |
| Compound 1 1 µM | - | | 31.6 (10.3) | | 12.3 (0.9) | 14.5 (1.7) |
| Compound 2 1 µM | 44.2 (0.3) | | | 26.7 (5.8) | | |
| Compound 1 2 µM | - | | 39.7 (9.8) | | 13.6(1.4) | 19.6 (1.9) |
| Compound 2 2 µM | 41.2 (0.4) | | | 38.5 (7.3) | | |
| Compound 1 5 µM | | 37.8 (16.1) | 52.2 (17.5) | 53.5 (23.1) | 23.3 (1.1) | 15.0 (2.0) |
| Compound 2 5 µM | 60.8 (18.6) | | | 87.8 (4.2) | | |

### Example 16

We also tested the ability of compound 1 to suppress proliferation of cancer cells by an alternative approach via the real time monitoring with a cellular analyser Juli FL. In brief, MCF7 cells were seeded in a 6-well plate the day before to reach approximately 5% confluence on the following day. Next day, the camera of the instrument was focused on a particular field with approximately 5% confluence and cells were continuously monitored, recording the confluence and visual appearance every 30 minutes for 48 hrs. Values were then expressed either as simple confluence at the end of the experiment or as slope of the line representing the confluence during a given period of time (Table 5). It is clear from the collected data that we do see a significant inhibition of cellular proliferation at a 30 nM concentration of compound 1.

**Table 5 The effect of compound 1 on cellular proliferation in malignant MCF7 cells, measured with continuous monitoring of cell confuence via Juli FL. Data represent mean values of cellular confluence or mean values of slope of the line that measures the relative number of cells in relation to time while numbers in brackets represent standard deviations in both cases.**

| Concentration | MCF7 | | |
|---|---|---|---|
| | Slope 0-22 h | Slope 22-48 h | Final % confluence |
| 0 µM | 0.536 (0.009) | 0.938 (0.005) | 34.49 (8.7) |
| 30 nM | 0.258 (0.005) | 0.404 (0.005) | 15.01 (3.4) |

### Example 17

Application of compound 1 leads to marked inhibition of cellular respiration as evidenced by the OCR measurements using the Seahorse instrument (Table 6). For Seahorse analysis, 20.000 cells were seeded in 96 well plates and added immediately before measurement (addition, Table 6) or incubated with compound 1 for 1 hour (preincubation, Table 7). Afterwards, oxygen consumption rate (OCR) was measured in three cycles of 3 minutes preceded by 3 minutes of mixing, using a Seahorse XFe96 analyzer (Agilent). Results express the mean values of the three cycles. The data show that both conditions lead to significant suppression of OCR.

**Table 6 The effect of immediate addition of compound 1 on oxygen consumption rate (OCR) in MCF7 cells as measured by the Seahorse instrument. Numbers represent mean Oxygen consumption rate (OCR; pmol^{∗}min⁻¹ 10.000 cells⁻¹) while numbers in brackets represent standard deviations.**

| **Addition** | Control | 5 µM | 10 µM |
|---|---|---|---|
| Basal | 71.5 (23.9) | 52.4 (9.2) | 49.6 (9.6) |
| Proton-leak | 20.1 (11.1) | 43.5 (7.6) | 37.3 (6.1) |
| Maximal | 80.4 (26.4) | 29.7 (5.8) | 26.3 (5.4) |

**Table 7 The effect of compound 1 on oxygen consumption rate (OCR) in MCF7 cells as measured by the Seahorse instrument after a 1h preincubation with compound 1. Numbers represent mean Oxygen consumption rate (OCR; pmol^{∗}min⁻¹ 10.000 cells⁻¹) while numbers in brackets represent standard deviations.**

| **1h preincubation** | Control | 5 µM | 10 µM |
|---|---|---|---|
| Basal | 77.1 (23.8) | 39.9 (5.8) | 30.9 (3.1) |
| Proton-leak | 19.2 (10.2) | 33.1 (4.7) | 25.9 (3.1) |
| Maximal | 78.9 (22.2) | 20.7 (4.5) | 17.4 (3.8) |

### Example 18

The effect of compound 1 on generation of mitochondrial reactive oxygen species (ROS) was evaluated in MCF7 and MDA-MB-231 cells using a specific mitochondrially targeted probe mitoSOX (ThermoFisher Scientific) that measures mostly mitochondrial superoxide and upon binding becomes fluorescent. Cells treated with compound 1 for the described time interval were stained with the probe and then analyzed via Flow cytometry. The data show that compound 1 is able to induce mitochondrial ROS.

**Table 8 MitoSOX probe measurement represent mean fluorescence values while numbers in brackets represent standard deviations.**

| Time | Compound 1 | |
|---|---|---|
| | MCF7 | MDA-MB-231 |
| 0 min | 100 (33.3) | 100 (16.2) |
| 5 min | 176.5 (15.0) | 134.7 (36.9) |
| 10 min | 177.6 (26.1) | 196.7 (41.5) |
| 15 min | 191.6 (47.0) | 284.7 (103.2) |
| 30 min | 230.8 (85.2) | 379.1 (76.9) |
| 60 min | 235.9 (99.3) | 439.1 (83.0) |
| 120 min | 171.1 (17.6) | 588.2 (14.11) |
| 180 min | 165.4 (8.9) | 560.8 (27.5) |
| 240 min | 207.7 (18.5) | 612.5 (37.5) |

### Example 19

Since compound 1 is an iron chelator, we have evaluated the effect of pre-loading compound 1 with iron at a 2:1 ratio and then applying it to the cells, followed by measurement of cell death using the Annexin V/PI staining as in example 4. Such pretreatment visibly reduced the extent of cell death induced by this chelator, confirming that the iron-chelating properties are partially responsible for its action in the induction of cell death but probably other mechanism(s) participate in the death inducing effect as well (Table 9).

**Table 9: The effect of iron preloading on the efficacy of compound 1 to kill cancer cells (MCF7, MDA-MB-231) as measured by the AnnexinV/PI staining, performed identicaly as in example 4, after 48hrs of incubation. Numbers represent mean percentage of dead cells while numbers in brackets represent standard deviations.**

| Concentration | MCF7 | | MDA-MB-231 | |
|---|---|---|---|---|
| | Alone | + Fe⁺³ | alone | + Fe⁺³ |
| 0 µM | 5.4 (2.3) | 11.1 (2.0) | 6.7 (1.6) | 4.9 (1.2) |
| 5 µM | 38.5 (8.8) | 24.3 (1.1) | 61.1 (18.8) | 15.9 (2.7) |
| 10 µM | 46.6 (7.8) | 25.7 (2.5) | 88.2 (5.8) | 22.4 (6.1) |

### Example 20

To test whether compound 1 is active against resistant cancer cells, we used tamoxifen-resistant MCF7, T47D. The IC₅₀ values were again measured with the crystal violet assay performed as in example 12. We have documented that there is a significant difference in the ability of compound 1 to induce cytostatic and cytotoxic effects between the parental cells sensitive to tamoxifen and the resistant ones that grow even in the presence of tamoxifen (Table 11), yet the IC₅₀.values are still in the nM range, making this compound very efficient. This observation, together with the data suggesting that it is effective also in the triple negative breast cancer cell lines such as MBA-MB-231, show that compound 1 is active even in the hard-to-treat cancer subtypes.

**Table 10: The effect of compound 1 on cell death induction in malignant (MCF7, T47D) cells (parental) and their tamoxifen resistant counterparts (Tam5R) grown in the presence of 5 µM tamoxifen. Data represent mean IC₅₀ values obtained by the crystal violet assay while numbers in brackets represent standard deviations**

| IC₅₀ (µM) | MCF7 | | T47D | |
|---|---|---|---|---|
| | Control | Tam5R | Control | Tam5R |
| DFX | 10.9 (1.5) | 15.0 (2.4) | 60.8 (9.7) | 82.6 (19.0) |
| Compound 1 | 0.008 (0.003) | 0.038 (0.009) | 0.031 (0.015) | 0.112 (0.037) |

### Example 21

To test the effect of compound 1 on the viability and proliferation of other resistant cancer cell types, we utilized a model of cancer stem-like cells that can be cultivated *in vitro,* so-called "mammospheres". The cells grow as a 3D microtumors and exhibit resistance to many known chemotherapeutic drugs. In this assay MCF7 spheres were dissociated to single cells/ small spheres, seeded 10,000 cells per well on a special cell culture plastic that is non adherent in the Mammocult medium (Stem cell technologies) and compound 1 and compound 2 at 1 µM were added. This represents the non-cytotoxic concentration. Cells were left to grow and generate spheres for 140 hrs. The amount of cells was then measured by the Cell TiterGlo 3D reagent (Promega) that determines cell counts of 3D cultured cells as a function of their ATP content that is determined by a chemiluminescent assay. As can be seen, both compounds were potent in inhibiting the formation and growth of tumor spheres, suggesting that these compound are active against the cancer stem-like cells.

**Table 11: The effect of compound 1 and 2 on the formation and growth of MCF7 mammospheres that represent the model of cancer stem-like cells. Mammospheres were grown for 140 hrs in the presence or absence of compound 1 and 2 at 1 µM. The quantification of formed mammospheres is based on the Cell Titer Glo 3D assay that measures cellular ATP via luminescence. Data represent mean chemiluminescence ATP values normalized to control sample set as 100 while numbers in brackets represent standard deviations.**

| | Compound 1 | Compound 2 |
|---|---|---|
| 0 µM | 100 (29.68) | 100 (29.68) |
| 1 µM | 6.61 (1.91) | 2.82 (0.28) |

### Example 22

In order to define the effect on cellular level, we investigated the effect of compound 1 and 2 at non-cytotoxic concentrations of the drugs (1µM) on cell cycle. Cells were cultivated with the compounds for 24 and 48 hrs and then stained with Vybrant DyeCycle VioletReady Flow reagent (ThermoFisher Scientific) that binds to DNA and analyzed by Flow cytometry and Kaluza program to define the proportions of G1,S and G2/M phases of cell cycle. It is evident that both compounds lead to accumulation of cells in G1 phase if the cell cycle, which become more prominent at later time points (see Table 12, 13). This shows that the compounds are potent inhibitors of cell cycle, explaining the molecular mechanism of their cytostatic activity.

**Table 12 The effect of compound 1 and 2 on the cell cycle distribution. Cells were cultivated in the presence of 1 µM compounds for 24 hrs and then stained with the Vybrant DyeCycle VioletReady Flow reagent and analyzed by Flow cytometry. Proportions of cells in each of cell cycle phases was done using Kaluza software. Data represent mean values while numbers in brackets represent standard deviations.**

| Compound | MCF7 | | | MDA-MB-231 | | |
|---|---|---|---|---|---|---|
| | G1 | S | G2 | G1 | S | G2 |
| Control (no compound) | 60.6 (5.8) | 12.1 (6.0) | 27.2 (10.2) | 53.2 (3.9) | 18.7 (4.6) | 28.1 (7.0) |
| Compound 1 | 82.8 (1.6) | 4.0 (3.0) | 13.2 (1.4) | 64.6 (2.0) | 7.7 (4.3) | 27.7 (2.3) |
| Compound 2 | 74.0 (1.3) | 5.7 (1.7) | 20.3 (0.4) | 64.0 (7.7) | 7.3 (1.8) | 28.8 (8.0) |

**Table 13 The effect of compound 1 and 2 on the cell cycle distribution. Cells were cultivated in the presence of 1 µM compounds for 48 hrs and then stained with the Vybrant DyeCycle VioletReady Flow reagent and analyzed by Flow cytometry. Proportions of cells in each of cell cycle phases was done using Kaluza software. Data represent mean values while numbers in brackets represent standard deviations**

| Compound | MCF7 | | | MDA-MB-231 | | |
|---|---|---|---|---|---|---|
| | G1 | S | G2 | G1 | S | G2 |
| 0 µM | 59.5 (6.3) | 20.5 (4.1) | 20.0 (3.4) | 57.1 (2.0) | 15.8 (2.9) | 27.1 (5.0) |
| Compound 1 | 75.8 (2.1) | 11.9 (2.3) | 12.4 (2.5) | 65.0 (4.1) | 9.8 (0.8) | 25.2 (3.3) |
| Compound 2 | 77.5 (2.2) | 10.8 (4.8) | 11.7 (3.8) | 74.1 (3.7) | 7.4 (1.4) | 18.5 (5.1) |

## Claims

1. Compound of general formula I, 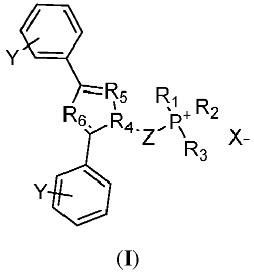 wherein
Z is a linear hydrocarbyl chain selected from alkylene, alkenylene and alkynylene, containing 2 to 20 carbon atoms, preferably 6 to 16 carbon atoms, more preferably 6 to 14 carbon atoms, even more preferably 8 to 12 carbon atoms, most preferably 10 carbon atoms, or preferably 8 to 16 or 10 to 15 carbons, wherein
- one or more carbon atoms (typically -CH₂- groups) in the hydrocarbyl chain may optionally be replaced by one or more 5-membered or 6-membered aromatic rings or heteroaromatic rings containing the heteroatoms O, S and/or N, said aromatic or heteroaromatic rings being preferably selected from phenylene, triazolylene or pyridylene, and/or
- one or more carbon atoms (typically -CH₂- groups) in the hydrocarbyl chain may optionally be replaced by one or more heteroatoms or heteroatom-containing moieties selected from O, S, NH, N-OH, and/or
- the hydrocarbyl chain atoms can be unsubstituted or substituted by one or more substituents selected independently from the group comprising C1-C4 alkyl, C1-C4 alkoxy, C1-C4 alkylthio, C1-C4 acyloxy, N(H or C1-C4 alkyl)₂ wherein alkyls are the same or different, phenyl, benzyl, OH, =O, SH, =S, =N-OH, F, Cl, Br, I;
each of R1, R2, R3 is independently selected from the group comprising C1-C10 alkyl, C6-C12 aryl, C6-C12-aryl-C1-C2-alkyl, C5-C12 heteroaryl, C3-C8 cycloalkyl, wherein each of R1, R2, R3 can optionally and independently from others be substituted by one or more substituents selected independently from the group comprising C1-C4 alkyl; C1-C4 alkoxy; C1-C4 alkylthio; N(H or C1-C4 alkyl)₂ wherein the alkyls are the same or different; OH; =O; SH; =S; =N-OH; F; Cl; Br; I;
each of R4, R5 and R6 is selected from CH and N, whereas at least two of R4, R5 and R6 are N;
X⁻ is a pharmaceutically acceptable anion;
Y is independently on each occurrence selected from the group comprising OH, SH, NH₂, F, Cl, Br, I and H.

2. The compound according to claim 1, wherein Z is selected from an alkylene; an alkylene wherein one carbon atom is replaced by a 5-membered or 6-membered aromatic ring or heteroaromatic ring containing the heteroatoms O, S and/or N; an alkylene wherein one carbon atom is replaced by phenylene, triazolylene or pyridylene; an alkylene wherein one carbon atom is replaced by NH; an alkylene wherein one carbon atom is replaced by phenylene, triazolylene or pyridylene, one carbon atom is replaced by NH and one carbon atom is substituted by =O, thus preferably forming the structure -(hetero)aryl-C(=O)-NH-;
wherein Z may optionally be further substituted.

3. The compound according to claim 1 or 2, wherein Y is H, OH, F or SH.

4. The compound according to any one of claims 1 to 3, wherein R6 is N, and at least one or R4 and R5 is also N.

5. The compound according to any one of claims 1 to 4 for use as a medicament.

6. The compound according to any one of claims 1 to 4 for use in a method of treatment of cancers.

7. The compound according to any one of claims 1 to 4 for use in a method of treatment of a cancer selected from breast, ovarian, prostate, GIT, hepatic, colorectal, pancreatic, mesothelioma, lung cancers and leukaemias.

8. A pharmaceutical composition containing at least one compound of general formula I according to any one of claims 1 to 4 and at least one pharmaceutically acceptable excipient, preferably selected from the group comprising carriers, solvents, fillers, binders, disintegrants, coatings, glidants, lubricants, preservatives, flavors, colorants.

9. A pharmaceutical composition comprising at least one compound of formula I according to any one of claims 1 to 4 and a metal, preferably selected from transition metals and metals of IIIA and IVA groups of the periodic table, more preferably the metal is iron.

10. The pharmaceutical composition according to claim 9 for use as a contrast agent for diagnostic, in particular for use in a method of *in vivo* visualisation of cancer.
